# EUROPEAN PATENT APPLICATION

(11) **EP 1 700 594 A1**
(43) Date of publication of application: **13.09.2006**
(21) Application number: 04805106.4
(22) Date of filing: 17.12.2004
(51) Int. Cl.: A61K 8/18

(54) **COSMETIC PRODUCT FOR SHAVING**

(30) Priority: 31.12.2003 ES 200303085
(71) Applicant: Picornell Garau, Antoni, Barcelona (ES); Healey, Elisabeth-Snow, Barcelona (ES)
(72) Inventor: PICORNELL GARAU, Antoni, E-08950 Esplugues De Llobregat (Barcelon (ES)
(74) Representative: Durán Moya, Carlos
(86) International application number: PCT/ES2004/000563
(87) International publication number: WO 2005/063181

(57) **Abstract**

The invention relates to a cosmetic product for shaving, which is **characterised in that** it forms a transparent gel comprising: 3-7% glycerine, 3-7% urea, 1-3% triethanolamine, 1-3% sodium cellulose carboxymethyl and 1-3% Tween 80, the remainder being purified water.

## Description

The invention is intended to disclose a cosmetic product for shaving which has substantial features of novelty and inventive step over the current state of the art.

As is known, there are a large number of cosmetic products on the market which soften the whiskers of the beard in order to permit shaving. Those products are of the soapy or non-soapy type, and may simply be in the form of gels which soften the beard, or of other types, all of which require the generation of foam by the shaving product which covers the face, either because the shaving product assumes the form of an aerosol on leaving the dispensing can or because the shaving product has inherent features of foam generation on being applied by the user himself to his face using his fingers.

In any case, the shaving products currently on the market form a covering for the whiskers which softens them and which permits the subsequent action of the razor blade, protecting the user's epidermis as far as possible against small wounds by the blades. However, all of these products currently on the market have in common the characteristic that, once applied to the face, they are not transparent and therefore the user has to shave without seeing the whiskers he wishes to cut.

US patent 2,833,693 discloses a transparent shaving gel based on a cellulose derivative as a gelling agent, such as sodium carboxymethylcellulose in a percentage of from 1.5% to 3.5%, from 5% to 10% of a polyol, such as a glycerol, propylene glycol or D-sorbitol as an agent promoting the gliding of the razor blade or a humectant, and also from 0.5% to 1.5% of a surfactant having beard-softening properties, such as Tween 20.

The disadvantage of that composition is that it cannot completely replace the whisker-softening effect which is achieved by means of the soap in conventional opaque shaving foams and creams and which has been described above, and it also has deficient properties in respect of the hydration of the skin.

US patent 20030170198 discloses a transparent shaving gel having from 15% to 20% of an agent for promoting the gliding of the blade or a humectant, such as, for example, glycerine, propylene glycol and/or glycereth-26, from 0.5% to 0.8% of a gelling agent, such as a carbomer, hydroxyethylcellulose or hydroxypropylcellulose, approximately from 2% to 5% of a skin conditioner/regenerator, such as Aloe vera gel, vitamin E and/or PEG 600, approximately from 0.5% to 0.8% of a neutraliser, such as triethanolamine, sodium hydroxide or potassium hydroxide, and approximately from 0.2% to 0.5% of a preservative, such as methyl paraben, propyl paraben and/or DMDM hydrantoine.

That composition is not able to solve the problems described above of replacing the effect of soap and lack of hydration, and therefore it includes a larger amount of humectant in order to promote the gliding of the blade and also regenerating agents in order to mitigate the adverse effects which shaving produces on the skin, but it is not capable of preventing these effects from being produced. On the other hand, the regenerating agents may have secondary effects, such as is the case, for example, of vitamin E, which may accumulate in the skin because it is liposoluble.

The present invention is intended precisely to overcome the disadvantage mentioned above, that is to say, it is to disclose a cosmetic shaving product which is transparent and which in no way reduces the effect of softening the beard and caring for the epidermis, in order to permit a good shave, but which also achieves the additional advantage that the user of this shaving product can see perfectly the whiskers of the beard once the shaving gel has been applied, so that the work of shaving is more efficient because the user, during the shaving operation, can assess the density of the whiskers, the direction thereof, the effects of the blade, etc., which enables the manual action of shaving to be adjusted in order to achieve more efficient effects.

According to the investigations and tests carried out by the Applicant, the constituents which make up the shaving gel to which the present invention relates are, for a preferred embodiment, the following, the proportions being indicated by weight:

| | |
|---|---|
| glycerine | 3-7% |
| urea | 3-7% |
| triethanolamine | 1-3% |
| sodium carboxymethylcellulose | 1-3% |
| Tween 80 | 1-3% |
| purified water | remainder |

The composition of the shaving gel according to the present invention uses the weak organic base properties of triethanolamine (or the like) in an amount sufficient to replace the soap in conventional shaving creams. Moreover, the investigations and tests carried out by the Applicant have shown that this replacement permits the addition of urea (not soluble in soap) to the gel. Surprisingly, the combination of the triethanolamine and the urea produces a gel having physical characteristics enabling it to be used as a shaving product which has excellent properties in respect of hydration of the skin and in which there is also obtained the synergic effect that the skin-softening properties produced by the soap in conventional opaque shaving creams are satisfactorily replaced.

The shaving gel is obtained by mixing the indicated constituents using methods conventional in the cosmetics industry and adjusting the viscosity to a value which is preferably higher than 2 Pa.s, preferably higher than 4 Pa.s and likewise preferably not higher than 25 Pa.s.

The viscosity is determined at ambient temperature (25°C) and at atmospheric pressure (760 mm Hg) with a RHEOMAT 180 apparatus (METTLER) rotating at 200 rpm. In a preferred embodiment of the present invention, the compositions by weight are the following:

| | |
|---|---|
| glycerine | 5% |
| urea | 5% |
| triethanolamine | 2% |
| sodium carboxymethylcellulose | 2% |
| Tween 80 | 2% |
| purified water | 84% |

The tests used by the Applicant demonstrate that the new cosmetic shaving product has a high degree of efficiency in respect of the satisfactory moistening of the beard, which permits efficient shaving without producing wounds on the epidermis.

Some of the mentioned products which enable the cosmetic for shaving to which the present invention relates to be obtained could be replaced, if desired, by others having similar behaviour, such as, for example:
- As substitutes for carboxymethylcellulose there may be mentioned: methylcellulose, gum arabic, polyvinylpyrrolidone, carrageenin, hydroxypropylcellulose, gum tragacanth, carboxypolymethylene, agar gum, xanthan gum.
- As substitutes for triethanolamine there may be indicated: monoethanolamine and diethanolamine.
- As substitutes for Tween 80 there may be mentioned: (polysorbate 80), (sorbitan polyester), polysorbate 20, polysorbate 60, sorbitan 20 ester, sorbitan 60 ester, sorbitan 80 ester.
- As substitutes for glycerine there may be mentioned: liquid paraffin, sweet almond oil, coconut oil, olive oil, palm oil, sunflower oil, corn oil, jojoba oil, avocado oil.

As will be appreciated, the compositions indicated are purely of a non-limiting exemplary nature so that the person skilled in the art, starting from the subject-matter disclosed in the present Application, could introduce a large number of variants by providing equivalents of the constituents provided for in the present invention, which would be included in the scope of the present invention defined by the following claims.

## Claims

1. Cosmetic product for shaving, constituted by a transparent gel which contains a gelling agent, an agent which promotes the gliding of the blade or a humectant and a surfactant, **characterised in that** it includes the following constituents:
| | |
|---|---|
| glycerine | 3-7% |
| urea | 3-7% |
| triethanolamine | 1-3% |
| sodium carboxymethylcellulose | 1-3% |
| Tween 80 | 1-3% |
| purified water | remainder |
the percentages being expressed by weight.

2. Cosmetic product for shaving according to claim 1, **characterised in that** the constituents of the transparent gel are preferably the following:
| | |
|---|---|
| glycerine | 5% |
| urea | 5% |
| triethanolamine | 2% |
| sodium carboxymethylcellulose | 2% |
| Tween 80 | 2% |
| purified water | 84% |
the proportions being indicated by weight.

3. Cosmetic product for shaving according to claim 1,
**characterised in that** it is possible to use as a replacement for glycerine: liquid paraffin, sweet almond oil, coconut oil, olive oil, palm oil, sunflower oil, corn oil, jojoba oil, avocado oil.

4. Cosmetic product for shaving according to claim 1,
**characterised in that** it is possible to use as a replacement for triethanolamine: monoethanolamine and diethanolamine.

5. Cosmetic product for shaving according to claim 1, **characterised in that** it is possible to use instead of carboxymethylcellulose: methylcellulose, gum arabic, polyvinylpyrrolidone, carrageenin, hydroxypropylcellulose, gum tragacanth, carboxypolymethylene, agar gum, xanthan gum.

6. Cosmetic product for shaving according to claim 1,
**characterised in that** it is possible to use as a replacement for Tween 80: (polysorbate 80), (sorbitan polyester), polysorbate 20, polysorbate 60, sorbitan 20 ester, sorbitan 60 ester, sorbitan 80 ester.
